# EUROPÄISCHE PATENTANMELDUNG

(11) **EP 2 415 358 A2**
(43) Veröffentlichungstag der Anmeldung: **08.02.2012**
(21) Anmeldenummer: 11188258.5
(22) Anmeldetag: 08.11.2011
(51) Int. Cl.: A23L 1/00, A23L 1/03, A23L 1/22, A23L 1/236, A23L 1/29, A23L 1/30, A23L 2/60, A23P 1/04, A61K 9/14, A61K 9/48, A61K 36/28, A23L 1/302, A23L 1/305

(54) **Nahrungsergänzungsmittelpräparat, welches eine Steviakomponente enthält**

(30) Priorität: 20.05.2011 DE 102011050541; 12.07.2011 DE 102011051782; 30.09.2011 DE 102011054084
(71) Anmelder: PM-International AG, 1618 Luxembourg (LU)
(72) Erfinder: Die Erfindernennung liegt noch nicht vor
(74) Vertreter: Zech, Stefan Markus

(57) **Zusammenfassung**

Nahrungsergänzungsmittelpräparat, das insbesondere pulverförmig und/oder als Konzentrat vorliegt, umfassend eine Vielzahl von Nahrungsergänzungsstoffen, wie Vitamine und/oder Mineralstoffe, in einem vorbestimmten Verhältnis, sowie eine Steviakomponente.

## Beschreibung

Die Erfindung betrifft ein Nahrungsergänzungsmittelpräparat, das insbesondere pulverförmig und/oder als Konzentrat vorliegt, umfassend eine Vielzahl von Nahrungsergänzungsstoffen, wie Vitamine und/oder Mineralstoffe, in einem vorbestimmten Verhältnis.

Der Einsatz von Nahrungsergänzungsmittelpräparaten, beispielsweise im Sport oder zur Förderung der Gesundheit, ist bereits bekannt.

Nahrungsergänzungsmittelpräparate sind dazu bestimmt, die allgemeine Ernährung, bestehend beispielsweise aus kohlenhydrat- oder eiweißhaltigen Lebensmitteln, wie Nudeln, Fleisch oder dergleichen, oder auch Süßwaren, wie Kuchen oder Kekse, zu ergänzen. Im Gegensatz zu konventionellen Lebensmitteln und Getränken stellen Nahrungsergänzungsmittelpräparate üblicherweise ein Konzentrat von Nahrungsergänzungsstoffen oder sonstigen Stoffen mit ernährungsspezifischer physiologischer Wirkung dar. Sie werden gewöhnlich in dosierter Form, insbesondere in Form von Kapseln, Pastillen, Tabletten, Pillen, Brausetabletten und ähnlichen Darreichungsformen, Pulverbeuteln, Flüssigampullen, Flaschen mit Tropfeinsätzen und ähnlichen Darreichungsformen von Flüssigkeiten und Pulvern zur Aufnahme in abgemessenen Kleinmengen vertrieben. Typische Inhaltsstoffe sind Mineralstoffe, Vitamine und Antioxidantien.

Der Verbraucher derartiger Nahrungsergänzungsmittelpräparate ist üblicherweise besonders gesundheitsbewusst und achtet nicht nur ausschließlich auf den "Geschmack"**,** sondern auch auf mögliche Nebenwirkungen oder Risiken in Zusammenhang mit Inhaltsstoffen. Besonders wichtig ist diesem Verbraucher dabei, nicht einer unübersehbaren Vielzahl von Nahrungsergänzungsstoffen gegenübergestellt zu werden, sondern in nachvollziehbarer Weise die Zusammensetzung des Nahrungsergänzungsmittelpräparates erfassen und analysieren zu können. Insofern ist es durchaus erwünscht, die Menge an Nahrungsergänzungsstoffen möglichst gering zu halten, ohne jedoch Einbußen bezüglich der gewünschten Nahrungsergänzungsmittelwirksamkeit hinnehmen zu müssen.

Bei Nahrungsergänzungsmittelpräparaten besteht ein weiteres Problem üblicherweise darin, dass die Nahrungsergänzungsstoffe entweder geschmacklos sind oder einen nur geringen Eigengeschmack aufweisen. Um die Akzeptanz beim Verbraucher zu erhöhen, muss daher das Nahrungsergänzungsmittelpräparat derart zusammengestellt werden, dass der Verbraucher auch einen gewissen Genuss empfindet, wenn er das Nahrungsergänzungsmittelpräparat anwendet. In diesem Zusammenhang ist es beispielsweise üblich, dem Nahrungsergänzungsmittelpräparat Zucker oder künstliche Süßstoffe, wie beispielsweise Saccharin, zuzusetzen. Zucker hat dabei den Nachteil, dass er eine vergleichsweise hohe Kalorienzahl aufweist und von daher oftmals aufgrund des Risikos einer Gewichtszunahme oder sonstiger gesundheitlicher Nachteile unerwünscht ist. Künstliche Süßstoffe sind insbesondere beim hier angesprochenen Anwender des Nahrungsergänzungsmittelpräparates nicht besonders beliebt, da sie als "unnatürlich" bzw. "unangenehm" hinsichtlich ihres Geschmacks empfunden werden und als "chemische" Komponente unerwünscht sind. In jedem Fall gelten Zucker und künstliche Süßstoffe nicht als gesundheitsfördernd.

Es ist daher Aufgabe der vorliegenden Erfindung, ein Nahrungsergänzungsmittelpräparat vorzuschlagen, das sich nicht nur durch einen angenehmen Geschmack auszeichnet, sondern möglichst auch mit möglichst wenigen Inhaltsstoffen eine gesundheitsfördernde Wirkung hat.

Diese Aufgabe wird durch ein Nahrungsergänzungsmittelpräparat nach Anspruch 1 sowie eine Verwendung nach Anspruch 22 gelöst.

Insbesondere wird die Aufgabe durch ein Nahrungsergänzungsmittelpräparat, das insbesondere pulverförmig und/oder als Konzentrat vorliegt, gelöst, umfassend eine Vielzahl von Nahrungsergänzungsstoffen, wie Vitamine und/oder Mineralstoffe, in einem vorbestimmten Verhältnis, sowie eine Steviakomponente.

Unter Steviakomponente soll im Folgenden eine Komponente verstanden werden, die zumindest teilweise aus der Pflanze "Stevia Rebaudiana" gewonnen ist bzw. gewonnen werden kann.

Ein Kerngedanke der Erfindung liegt somit darin, eine Steviakomponente in einem Nahrungsergänzungsmittelpräparat zu verwenden. Insofern wird auch die Verwendung einer Steviakomponente in einem Nahrungsergänzungsmittel, insbesondere als Süßungsmittel und/oder Antioxidans, unabhängig vorgeschlagen.

Der Einsatz einer Steviakomponente in Nahrungsergänzungsmittelpräparaten führt in synergistischer Weise dazu, dass das Nahrungsergänzungsmittelpräparat aufgrund der Süßungswirkung der Steviakomponente angenehm bei der Anwendung und gleichzeitig durch seine antioxidative Wirkung der Gesundheit förderlich ist. Es wird daher mit nur einer Komponente zwei wesentlichen Aspekten eines Nahrungsergänzungsmittelpräparates, nämlich dass dieses einerseits geschmacklich angenehm sein soll und der Gesundheit förderlich sein soll, Rechnung getragen. Die Zusammensetzung des Nahrungsergänzungsmittelpräparates wird dabei nicht weiter verkompliziert. Die Verwendung der bespielsweise aus der WO 2011/046233 A1 bekannten Süßungswirkung von Stevia bzw. aus Stevia gewonnenem Rebaudiosid ist im Bereich von Speisen, wie Kuchen, Keksen, Eiscreme, Softdrinks oder Schokolade bekannt. Durch die Verwendung von Stevia in einem Nahrungsergänzungsmittelpräparat kann jedoch auch noch auf besonders vorteilhafte Weise die antioxidative Wirkung von Stevia genutzt werden.

Als Steviakomponente können getrocknete Pflanzenteile der Stevia Rebaudiana, wie beispielsweise deren Blätter, und/oder ein Stevia-Extrakt, also ein Extrakt, welches aus der Stevia Rebaudiana gewonnen wird, und/oder ein Steviosid und/oder Steviobiosid und/oder Rebaudiosid und/oder Dulcosid, vorliegen. Insbesondere bei der Verwendung eines Stevia-Extraktes hat sich eine vorteilhafte antioxidative Wirkung gezeigt.

Vorzugsweise ist die Steviakomponente maskiert, weiter vorzugsweise durch Aufsprühen und/oder eine Verkapselung mit einem Maskierungsmittel. Derartige Maskierungstechniken sind im Stand der Technik bekannt, wobei beispielhaft auf die WO 2011/048616 A2 verwiesen wird, in der eine Maskierung mit Kochsalz vorgeschlagen wird. Hinsichtlich der Maskierung wird hiermit auf die WO 2011/048616 A2 verwiesen. Im Zusammenhang mit Nahrungsergänzungsmittelpräparaten, die normalerweise eine Vielzahl von geschmacklosen oder nur wenig Geschmack aufweisenden Komponenten enthalten, ist eine Maskierung des üblicherweise bitterlakritzartigen Beigeschmacks von Stevia von Vorteil. Insofern dient diese Maskierung ebenfalls der Verwirklichung einer Doppelfunktion von angenehmem Geschmack und antioxidativer Wirkung.

Die Steviakomponente liegt vorteilhafterweise in Form von insbesondere mizellierten Partikeln, beispielsweise als Pulver, vor. Dadurch wird in synergistischer Weise das Geschmacksempfinden verbessert, da die Geschmackskurve angenehm nach hinten ausklingt und gleichzeitig die Steviakomponente als antioxidierende Komponente vom Körper einfach resorbiert werden kann. Besonders bevorzugt ist dabei eine mizellierte Form, bei der die Steviakomponente in Partikelgrößen von maximal 200 nm vorliegt, was eine besonders gute Aufnahme der Steviakomponente durch den menschlichen Körper ermöglicht, und somit die Wirksamkeit der Steviakomponente steigert.

Vorzugsweise enthält das Nahrungsergänzungsmittelpräparat eine koffeinhaltige Substanz, insbesondere Guarana und/oder Niacin und andere B-Vitamine und/oder mindestens eine als Radikalfänger wirkende Substanz und/oder getrocknete Braunalgen oder Braunalgenextrakte, wahlweise in Form von deren Salzen. Ein derartiges Nahrungsergänzungsmittelpräparat hat einige positive, gewünschte Wirkungen bei Verabreichung in angemessener Dosis, wie beispielsweise Erhöhung der körperlichen und geistigen Leistungsbereitschaft, Förderung der Durchblutung, etc. Weiterhin besitzt ein derartiges Nahrungsergänzungsmittelpräparat gute Sauerstofftransporteigenschaften und zeichnet sich weiter dadurch aus, dass Zellbarrieren hervorragend durchdrungen werden und so Sauerstoff nicht nur transportiert, sondern den Zellen selbst zusätzlich Sauerstoff zugeführt wird. Die Algensubstanz, umfassend getrocknete Braunalgen oder Braunalgenextrakte verstärkt in synergistischer Weise die Wirkung der weiterhin enthaltenen Substanzen. Guarana als koffeinhaltige Substanz wird bevorzugt, da es eine im Vergleich zu Koffein längere Wirkungszeit aufweist und damit der erwünschte Wachhalteeffekt länger anhält. Als Radikalfänger wirkende Substanzen kommen antioxidativ wirkende Vitamine, wie beispielsweise Vitamin C, in Betracht. Diese antioxidativ wirkenden Vitamine ergänzen noch besonders vorteilhaft die antioxidative Wirkung der Steviakomponente. In einem bevorzugten Ausführungsbeispiel beträgt der Mindestgewichtsanteil der getrockneten Braunalgen oder der Braunalgenextrakte mindestens 0,0001 Gewichtsprozent, bezogen auf die durch die oben angegebenen Inhaltsstoffe definierte Mischung. Eine Höchstgrenze kann bei 2 Gewichtsprozent liegen. Die oben genannte Konzentrationsangabe für getrocknete Braunalgen oder der Braunalgenextrakte ist bezogen auf die zuvor angegebenen Substanzen.

Insgesamt kann das erfindungsgemäße Nahrungsergänzungsmittelpräparat vorteilhafterweise noch einen Anteil an Zusatzstoffen, wie Träger- oder Geschmacksstoffe aufweisen. Die Trägerstoffe können auch zu einer Resorptionsverbesserung im Körper bewirken. Die Konzentration dieser Zusatzstoffe liegt vorteilhafterweise im Bereich zwischen 20 und 95 Gewichtsprozent des Nahrungsergänzungsmittelpräparates, weiter vorzugsweise im Bereicht zwischen 60 und 95 Gewichtsprozent. In der bevorzugten Ausführungsform liegt das Nahrungsergänzungsmittelpräparat in pulvriger Form zur Herstellung einer wässrigen Lösung vor, wobei eine Dosierung von 1 bis 20 g auf ein Glas (0,2 I bis 1,0 I) Wasser zweckmäßig erscheint. Alternativ kann die Mischung aber auch in flüssiger oder fester Form, beispielsweise als Tablette, vorliegen.

Das Nahrungsergänzungsmittelpräparat kann als Mittel zur Erhöhung/Konzentration des O₂-Antransportes im menschlichen oder tierischen Zellsystem verwendet werden. Wenn getrocknete Braunalgen oder Braunalgenextrakte mit einer koffeinhaltigen Substanz zum Einsatz kommen, wird den Zellen wesentlich mehr Sauerstoff zugeführt, was sich in konkreten Experimenten in Steigerungen der körperlichen und geistigen Leistungsfähigkeit nachweisen lässt.

Eine weitere Wirkung des erfindungsgemäßen Nahrungsergänzungsmittelpräparates besteht darin, dass die Durchblutung ganz merklich gefördert wird. Die Wirkung des Nahrungsergänzungsmittelpräparates setzt nach oraler Einnahme in etwa nach zehn Minuten ein und wird von den Probanden als Wärmesensation auf bestimmten Hautpartien empfunden und lässt sich mit thermografischen Bildaufnahmeverfahren, insbesondere in einer thermografischen Bildserie belegen. Die thermografischen Untersuchungen belegen, dass die Wirkungen der Mischung auf einige Körperpartien einschließlich des Kopfes erstrecken. In einer ersten Studie konnte belegt werden, dass die erfindungsgemäße Mischung, wie bereits zuvor eine der Einzelkomponenten dieser Mischung, die einer Stichprobe von Probanden oral verabreicht wurde, einen signifikanten Einfluss auf die körperliche Leistungsfähigkeit ausübt, was sich an der signifikanten Erhöhung des RQ's (Respiratorischer Quotient) bestätigen ließ. Ein Reaktionstest zeigte, dass die Vigilanz der Probanden durch die Mischung gesteigert werden konnte.

Vorzugsweise enthält das Nahrungsergänzungsmittelpräparat probiotische Kulturen mit einem vorbestimmten Anteil an probiotischen Milchsäurebakterien sowie weiter vorzugsweise ein Gewürzextrakt nach dem ayurvedischen Prinzip (Maricha) als Alkaloid. Es ist bereits bei Nahrungsergänzungsmittelpräparaten, beispielsweise bei der Verabreichung von Beta-Carotin, Selen, Vitamin B₆, Vitamin C, Vitamin A, Vitamin A + D festgestellt worden, dass die Beimengung eines Gewürzextraktes nach dem ayurvedischen Prinzip eine resorptionsverstärkende Wirkung auslöst. Gewürzextrakt nach dem ayurvedischen Prinzip scheint hier als eine Art Katalysator zu wirken, um die biologischen Barrieren, insbesondere Darmschleimhaut und Zellwände, besser zu durchdringen. Es hat sich dabei in überraschender Weise gezeigt, dass die Wirkungsweise von Gewürzextrakten nach dem ayurvedischen Prinzip noch unterstützt werden kann, wenn durch den Einsatz von probiotischen Kulturen eine insgesamt positive Darmflora und damit eine günstige Situation im Darmtrakt, insbesondere an der Darmschleimhaut, geschaffen werden kann. Die Darmschleimhaut kann durch die probiotischen Kulturen für die besondere Wirkung des Gewürzextraktes nach dem ayurvedischen Prinzip zweckmäßig vorbereitet werden.

In einer vorteilhaften Ausgestaltung enthält das Nahrungsergänzungsmittelpräparat weiterhin mindestens eine als Radikalfänger wirkende Substanz, insbesondere umfassend Bioflavonoide. Insbesondere, wenn probiotische Kulturen einerseits und Gewürzextrakt nach dem ayurvedischen Prinzip andererseits beigefügt sind, kann der Radikalfänger zielgerichtet an die vorgesehenen Wirkungsorte transportiert werden. Liegt als Radikalfänger bzw. Antioxidans eine Steviakomponente vor, so kann auch diese besonders günstig transportiert werden.

Bei der Verwendung von probiotischen Kulturen ist es vorteilhaft, wenn die Kulturen Fructooligosaccharide und/oder Inulin enthalten.

Weiterhin ist es in zweckmäßiger Weise vorgesehen, dem Nahrungsergänzungsmittelpräparat eine Mehrzahl verschiedener löslicher und/oder unlöslicher Ballaststoffe beizumischen.

In einer konkreten Ausgestaltung kann weiterhin eine Mehrzahl verschiedener pflanzlicher Verdauungsenzyme in dem Nahrungsergänzungsmittelpräparat enthalten sein.

Die Resorption von Selen wird durch Gewürzextrakt nach dem ayurvedischen Prinzip gesteigert. In einer vorteilhaften Ausgestaltung wird dem Nahrungsergänzungsmittelpräparat auch Selen beigemischt, wobei die erreichte Resorption durch die synergetische Wirkungsweise von probiotischen Kulturen einerseits und Gewürzextrakt nach dem ayurvedischen Prinzip andererseits nochmals gesteigert ist.

In einer konkreten Ausgestaltung ist das Gewürzextrakt nach dem ayurvedischen Prinzip in einer Konzentration enthalten, die einer Tagesdosis von 1 bis 10 mg, vorzugsweise 3 bis 7 mg, besonders bevorzugt etwa 5 mg pro Tag enspricht.

Bevorzugtermaßen sind, bezogen auf eine Tagesdosis von 5 mg, in dem erfindungsgemäßen Nahrungsergänzungsmittelpräparat probiotische Kulturen mit einer Keimmenge von 10⁷ bis 10¹¹ Keimen, vorzugsweise 10⁸ bis 10¹⁰ Keimen, insbesondere von etwa 10⁹ Keimen, enthalten. Die hier angegebenen Keimmengen beziehen sich auf die Anzahl lebender bzw. lebensfähiger Keime zum Zeitpunkt der Beimischung in das Nahrungsergänzungsmittelpräparat. In dem Nahrungsergänzungsmittelpräparat können weiterhin Zuschläge wie Träger oder Geschmacksstoffe enthalten sein. In einer konkreten Ausgestaltung beläuft sich der Gewichtsanteil der Zuschläge am Präparat in einem Bereich zwischen 20 und 95 Gew.%, vorzugsweise im Bereich zwischen 60 und 95 Gew.%. Das Präparat kann in flüssiger Form, in Form einer Emulsion oder in einer anderen beliebigen Form vorliegen. Bevorzugtermassen ist das Präparat in pulvriger Form zur Herstellung einer wässrigen Lösung bereitgestellt.

Vorzugsweise kommt in dem Nahrungsergänzungsmittelpräparat ein kapillardilatives Agens, insbesondere Nicotinsäure, zur Erzielung einer Kapillardilatation im Intestinaltrakt zum Einsatz. Gerade in Kombination mit probiotischen Kulturen mit einem vorbestimmten Anteil an lebenden probiotischen Milchsäurebakterien und einem thermogenetisch-lipolytisch-resorptionssteigernden Agens, insbesondere Pfefferextrakt zur Verstärkung der resorptiven Prozesse im Darm, können probiotische Kulturen im Darm bzw. im Intestinaltrakt mit noch höherer Effizienz ihre Wirkung entfalten. Das im Piper nigrum enthaltene Alkaloid Piperin als Pfefferextrakt zeigt einen thermogenetischen und/oder lipolytischen und/oder resorptionsverstärkenden Effekt. Das kapillardilative Agens, insbesondere Nicotinsäure, bewirkt eine Kapillardilatation auch im Intestinaltrakt mit dadurch resorptionsfördernder Wirkung und verbessert die Sauerstoffaufnahme sowie die Stoffwechselendproduktabgabe.

In der Kombination mit probiotischen Kulturen und thermogenetisch-lipolytisch-resorptionssteigernden Agens, insbesondere Pfefferextrakt, und dem kapillardilativen Agens, insbesondere der Nicotinsäure, verstärkt diese die Wirksamkeit der probiotischen Kulturen, insbesondere deren Aktivität und Adhäsion im Intestinalbereich.

Eine ausgewogene Wirkung der probiotischen Kulturen ergibt sich, wenn die probiotischen Kulturen mindestens zwei, vorzugsweise mindestens drei, besonders bevorzugt mindestens vier verschiedene Kulturen (Keimstämme) umfassen. Die probiotischen Kulturen ergänzen sich dabei teilweise und können sich in gewissen Konstellationen auch gegenseitig unterstützen. Die probiotischen Kulturen können als eine einzige Kultur oder in Kombination aus zwei oder mehr Kulturen aus der Gruppe umfassend Lactobacillus acidophilus, Lactobacillus delbrückii, insbesondere Lactobacillus bulgaricus, Bifidobakterien, Streptococcus thermophilus ausgewählt sein. Mit diesen konkreten Kulturen haben sich gerade bei der Beimengung eines thermogenetisch-lipolytisch-resorptionssteigernden Agens, insbesondere Pfefferextrakt und eines kapillardilatives Agens, insbesondere Nikotinsäure gute Ergebnisse erzielen lassen. Bevorzugtermassen sind dem Pulver weiterhin Prebiotics, insbesondere Inulin und/oder Lactose zur Förderung der probiotischen Kulturen zugesetzt. Die Prebiotics dienen als Hilfs- und Nährstoffe für die probiotischen Kulturen und fördern deren Antransport in den Intestinaltrakt sowie deren Gedeihen und Aktivität im Intestinaltrakt. In einer bevorzugten Ausgestaltung umfasst das Pulver weiterhin Molkeeiweiss insbesondere mit einem hohen Anteil von B-Vitaminen. B-Vitamine, insbesondere Vitamin B1, B2, B6 und B12 sind für zahlreiche Körperfunktionen lebensnotwendig; eine Unterversorgung führt zu Mangelerscheinungen.

In einer weiteren Ausgestaltung enthält das Pulver Calcium und/oder Phosphor, was unter anderem dem Knochenaufbau dient. Das Pulver kann in einer weiteren Ausgestaltung eine vorbestimmte Menge an Oligofructose enthalten, einerseits als Ballaststoff, zum anderen, um die probiotischen Kulturen möglichst gut durch das Säuremilieu des Magens zu schleusen.

In einer besonders bevorzugten Ausgestaltung umfasst das Pulver gemäß der vorliegenden Erfindung eine Anzahl an probiotischen Keimen bezogen auf die fertige Joghurtspeise von über 10 Mio./g, vorzugsweise von über 50 Mio./g, besonders bevorzugt von über 100 Mio/g.

Eine besonders zweckmässige Konzentration des thermogenetisch-lipolytisch-resorptionssteigernden Agens, insbesondere des Pfefferextrakts liegt bezogen auf 100 ml fertige Joghurtspeise in einem Bereich von 0,1 mg bis 10 mg, vorzugsweise bei etwa 1,0 mg.

Eine besonders zweckmäßige Konzentration des kapillardilatives Agens, insbesondere der Nikotinsäure liegt bezogen auf 100 ml fertige Joghurtspeise in einem Bereich von 2 mg bis 20 mg, vorzugsweise bei etwa 10 mg. Der schwach saure Geschmack führt zu keiner unangenehmen Geschmacksveränderung der mit dem Pulver herstellbaren Joghurtspeise.

In einer besonders bevorzugten Ausgestaltung ist das Pulver zum Ansatz einer probiotischen Joghurtspeise in einem Licht- und Sauerstoffdurchtritt hemmenden Folienbeutel untergebracht. Hierdurch wird eine lange Haltbarkeit, insbesondere die Aufrechterhaltung hoher Aktivität der gefriergetrockneten probiotischen Kulturen sichergestellt.

Erfindungsgemäß wird weiterhin auch eine probiotische Joghurtspeise beansprucht, die aus einem Ansatz aus dem beschriebenen Pulver enthaltend eine Steviakomponente, insbesondere homogenisierte H-Milch nach einer Generierungszeit von einigen Stunden hergestellt wird. Hierzu kann ein besonderes Bereitungssystem eingesetzt werden, das auf das erfindungsgemäße Pulver abgestimmt ist. Dieses Bereitungssystem besteht aus einem Innentopf, in dem das Pulver vorzugsweise mit H-Milch in einer bestimmten Menge, vorzugsweise einem Liter, angerührt wird. In den Außentopf wird kochendes Wasser in einer vorbestimmten Menge gefüllt. Der den Ansatz enthaltende Innentopf wird durch den mit kochendem Wasser befüllten Außentopf erwärmt, wobei durch den Wärmegradienten im Innentopf ein Konvektionseffekt entsteht, der verhindert, dass die temperaturempfindlichen probiotischen Kulturen in den heissen Randbereichen des Innentopfes Schaden nehmen. Durch den Konvektionseffekt erhalten alle Keime bei der vorzugsweise acht bis 10 Stunden betragenden Generierungszeit ihr Temperaturoptimum. Der fertige Joghurt ist über mindestens eine Woche im Kühlschrank haltbar bei Aufrechterhaltung einer hohen Keimzahl.

Die gesundheitlichen Wirkeffekte der probiotischen Kulturen werden durch den thermogenen und/oder lipolytischen und/oder resorptionsverstärkenden Effekt des thermogenetisch-lipolytisch-resorptionssteigernden Agens, insbesondere Pfefferextrakt sowie dem gefässdilativen Effekt mit Stoffwechselaktivierung des kapillardilatives Agens, insbesondere der Nikotinsäure in überraschender Weise noch entscheidend verstärkt.

Vorzugsweise enthält das Nahrungsergänzungsmittelpräparat die Mineralstoffe und Spurenelemente Vitamin D, Calcium, Magnesium, Selen, Kupfer, Zink und Chrom. Weiter vorzugsweise enthält das Nahrungsergänzungsmittelpräparat mindestens einen Katalysator und/oder Wirkstoff, vorzugsweise in Pulverform, der die Resorption der Mineralstoffe und/oder Spurenelemente steigert und/oder verbessert und/oder beschleunigt. Die Vorteile dieser Kombination liegen insbesondere in den verbesserten Resorptionseigenschaften (einschließlich des verbesserten Transports der Wirkstoffe an ihre Wirkstätte) des Nahrungsergänzungsmittelpräparates. Dadurch werden die Mineralstoffe und/oder Spurenelemente schneller in den Körper aufgenommen und können mit geringerem Zeitabstand zur Einnahme ihre Wirkung entfalten. Auch kann der Katalysator und/oder Wirkstoff die Wirksamkeit der Mineralstoffe und/oder Spurenelemente im Körper verbessern. Das Nahrungsergänzungsmittelpräparat eignet sich insbesondere dazu, Osteoporose und Übersäuerung entgegenzuwirken. Der Katalysator kann Schwarzpfefferextrakt umfassen oder daraus bestehen, insbesondere Piperin (eine chemische Variante ist 1-Piperoylpiperidin-C₁₇H₁₉NO₃) umfassen oder daraus bestehen.

Aus Schwarzem Pfeffer (piper nigrum) als Ausgangssubstanz lässt sich ein Piperin gewinnen, das als Katalysator und/oder Wirkstoff besonders effektiv ist.

Von Vorteil ist, wenn der Piperinanteil im Katalysator und/oder Wirkstoff im Bereich von 90 Prozent bis 100 Prozent liegt, insbesondere im Bereich von 92 bis 100 Prozent, vorzugsweise bei mindestens 95 Prozent. Eine Weiterbildung sieht vor, dass der Katalysator und/oder Wirkstoff vollständig oder zumindest weitestgehend in seiner molekularen Struktur, wie sie im Schwarzen Pfeffer vorliegt, belassen bleibt. Es soll sich dabei insbesondere um ein aus der Pflanze Schwarzer Pfeffer gewonnene Substanz und nicht um ein künstlich chemisch erzeugtes Produkt handeln. Eine spezielle Vermahlung kann die (zumindest weitestgehende) Aufrechterhaltung der natürlichen molekularen Struktur gewährleisten.

In diesem Zusammenhang hat es sich als vorteilhaft erwiesen, wenn das Nahrungsergänzungsmittelpräparat Magnesiumhydroxidcarbonat enthält. Viele der am Markt angebotenen Magnesiumverbindungen sind im Allgemeinen schwer resorbierbar. Üblich ist bislang vor allem die Verwendung von Magnesiumcarbonat. Demgegenüber führt die Verwendung von Magnesiumhydroxidcarbonat zu einer verbesserten Resorbierbarkeit des Magnesiums sowie zu einer Steigerung von dessen Wirksamkeit.

Zweckmäßigerweise enthält das Nahrungsergänzungsmittelpräparat je nach Ausführungsvariante Beta-Carotin (Provitamin A) und/oder Kalium und/oder Natrium und/oder Eisen. Eisen ist ein Additiv, das sich unter anderem bei starker Übersäuerung als vorteilhaft erwiesen hat. Besonders Hochleistungssportler und Frauen sollten daher auf ein Nahrungsergänzungsmittelpräparat mit Eisenanteil zurückgreifen, um die Hämoglobinsynthese sicherzustellen.

Eine bevorzugte Weiterbildung des Nahrungsmittelergänzungspräparat sieht vor, dass der Kaliumanteil im Präparat zwischen 0,15 und 35 Gew.% liegt, vorzugsweise zwischen 0,5 und 10 Gew.%, insbesondere bei 0,8 Gew.%, und/oder der Calciumanteil im Präparat zwischen 1,6 und 17 Gew.% liegt, vorzugsweise zwischen 2,5 und 10 Gew.%, insbesondere bei 4,5 Gew.% und/oder der Magnesiumanteil im Präparat zwischen 0,8 und 5,0 Gew.% liegt, vorzugsweise zwischen 2 und 3 Gew.%, insbesondere bei 2,2 Gew.%, und/oder der Zinkanteil im Präparat zwischen 0,015 und 0,2 Gew.% liegt, vorzugsweise zwischen 0,05 und 0,1 Gew.%, insbesondere bei 0,08 Gew.%, und/oder der Kupferanteil im Präparat zwischen 0,0015 und 0,017 Gew.% liegt, vorzugsweise zwischen 0,005 und 0,015 Gew.%, insbesondere bei 0,011 Gew.%, und/oder der Chromanteil im Präparat zwischen 0,00015 und 0,0017 Gew.% liegt, vorzugsweise zwischen 0,0002 und 0,0008 Gew.%, insbesondere bei 0,00028 Gew.%, und/oder der Selenanteil im Präparat zwischen 0,00015 und 0,0012 Gew.% liegt, vorzugsweise zwischen 0,0002 und 0,0006 Gew.%, insbesondere bei 0,00028 Gew.%, und/oder der Eisenanteil im Präparat bei nahezu 0 Gew.% oder zwischen 0,015 und 0,09 Gew.% liegt, vorzugsweise zwischen 0,03 und 0,09 Gew.%, insbesondere bei 0,08 Gew.%, und/oder der Vitamin-D-Anteil im Präparat zwischen 0,00001 und 0,00008 Gew.%, liegt, vorzugsweise zwischen 0,00002 und 0,00006 Gew.%, insbesondere bei 0,00004 Gew.%, und/oder der Anteil des Katalysators und/oder Wirkstoffs zur Resorptionsverbesserung, insbesondere der Piperinanteil, im Präparat zwischen 0,15 und 1,7 Gew.% liegt, vorzugsweise zwischen 0,4 und 1,2 Gew.%, insbesondere bei 0,8 Gew.% und/oder der Anteil der Steviakomponente zwischen 0,01 und 2 Gew.%, vorzugsweise zwischen 0,05 und 1%, weiter vorzugsweise zwischen 0,1 und 0,5 Gew. % liegt bzw. bei einem verwendeten Steviakonzentrat oder -extrakt einem solchen Anteil an getrocknetem Stevia entspricht (hinsichtlich der Süßungswirkung).

Zweckmäßige und bevorzugte Ausführungsvarianten sehen vor, dass das Nahrungsmittelergänzungspräparat in Pulverform vorliegt und/oder ausgebildet und bestimmt ist zur Herstellung einer wässrigen Lösung und/oder ausgebildet und bestimmt ist zur Herstellung eines Getränks, insbesondere eines Mineral-Basen-Gertränks. Als Getränk lässt sich das Präparat besonders einfach, flexibel und schnell einnehmen, wodurch eine schnelle Wirkung sichergestellt wird. Auch ermöglicht ein Getränk die problemlose und bequeme regelmäßige Verwendung, ohne die sich die gewünschten Wirkungen beim Menschen in der Regel nicht erzielen lassen.

Als zweckmäßig hat sich ferner ein Nahrungsmittelergänzungspräparat-Set erwiesen, das als Grundsubstanz ein erstes Nahrungsmittelergänzungspräparat nach der Erfindung, das kein Eisen enthält, und als Additiv ein zweites Nahrungsmittelergänzungspräparat nach der Erfindung, das Eisen enthält, umfasst. Die Grundsubstanz und/oder das Additiv können dabei für die eigenständige Verwendung geeignet sein. Insbesondere sind Grundsubstanz und Additiv zur Einstellung eines gewünschten Eisenanteils in beliebigem Verhältnis mischbar. Die Bedeutung von Eisen für bestimmte Menschengruppen, insbesondere Sportler und/oder Frauen, wurde bereits obenstehend dargelegt. Das Nahrungsmittelergänzungspräparat-Set ermöglicht es dem jeweiligen Verwender, den Eisenanteil entsprechend seinen individuellen Bedürfnissen durch entsprechendes Mischen von Grundsubstanz und Additiv einzustellen, so dass ihm dadurch ein optimal zusammengesetztes Präparat zur Verfügung steht.

Nachfolgend werden die charakteristischen Werte eines Ausführungsbeispiels des Nahrungsmittelergänzungspräparats gemäß der Erfindung angeführt.

Ausgehend von einer Nahrungsmittelergänzungspräparat-Portion von 6 g (Gramm), was der einzunehmenden Tagesmenge entspricht, gibt folgende Tabelle typische Mengenwerte der einzelnen Mineralstoffe bzw. Spurenelemente innerhalb dieser Portion an. Das heißt die Tagesportion von 6 g enthält 50 mg (Milligramm) Kalium, 270 mg Calcium, usw. Ferner sind die Grenzen angegeben, innerhalb derer die jeweilige Substanz variiert werden kann. Neben den angegebenen Substanzen sind in der Portion noch weitere Substanzen enthalten, die beispielsweise der Aromatisierung und/oder der Löslichkeit des Nahrungsmittelergänzungspräparats dienen. Dadurch werden eine guten Löslichkeit und eine geschmackliche Akzeptanz des Nahrungsmittelergänzungspräparats erreicht.

| Mineralstoff bzw. Spurenelement | Menge in einer 6-Gramm-Portion | Grenzwerte |
|---|---|---|
| Kalium | 50 mg | 10 mg - 2.000 mg |
| Calcium | 270 mg | 100 mg - 1.000 mg |
| Magnesium | 134 mg | 50 mg - 300 mg |
| Zink | 5 mg | 1 mg - 11 mg |
| Kupfer | 670 µg | 100 µg - 1 mg |
| Chrom | 17 µg | 10 µg - 100 µg |
| Selen | 17 µg | 10 µg - 70 µg |
| Eisen | 5 mg | 1 mg - 5 mg |
| Vitamin D | 2,5 µg | 1 µg - 5 µg |

Ferner kann die Menge an Piperin pro 6-Gramm-Portion typischerweise bei 0,1 mg bis 10 mg, vorzugsweise bei 0,3 mg bis 2 mg, insbesondere bei 0,5 mg liegen.

Ferner kann die Menge an Stevia pro 6-Gramm-Portion typischerweise bei 0,6 mg bis 120 mg, vorzugsweise bei 3,0 mg bis 60 mg, insbesondere bei 6 mg liegen.

In einem Nahrungsmittelergänzungspräparat-Set entspricht die Grundsubstanz der obigen Zusammensetzung bis auf den Eisenwert, der bei 0 Gramm liegt. Das Additiv entspricht hingegen der vorstehenden Zusammensetzung einschließlich des Eisenwertes. Den individuell gewünschten Eisenwert kann der Verwender dann durch entsprechendes Mischen von Grundsubstanz und Additiv einstellen.

Das Nahrungsergänzungsmittelpräparat kann weiterhin Trigonella Foenum-Graecum L. (Bockshornkleesamen) und/oder Camelia sinsensis mit einem Anteil von Polyphenolen und Koffein und/oder ein Curcumaextrakt und/oder ein ayurvedisches Gewürzextrakt, insbesondere Pfefferextrakt, sowie Bitterorangenextrakt, enthalten. Trigonella Fuenum-Graecum L. (Bockshornkleesamen) kann dabei selbst verschiedene Wirkmechanismen hervorrufen, nämlich zunächst im Magen aufquellen und dadurch allein schon ein Gefühl der Sättigung erzeugen. Der Wirkmechanismus zwischen Magen und Gehirn beruht dabei auf entsprechenden nervalen Verbindungen und erfolgt über das vegetative Nervensystem.

Camelia sinsensis kann Polyphenole sowie Koffein in einem hohen Anteil bereitstellen. Die Polyphenole unterstützen die Thermogenese sowie die Fettverbrennung. Das Koffein kann als Initiator zur Fettverbrennung angesehen werden. Es unterstützt die Energiebereitstellung und damit ebenfalls den Fettabbau.

Curcumaextrakt kann eine Appetitzügelung bewirken. Die Beimengung des ayurvedischen Gewürzextraktes, insbesondere Pfefferextraktes, stärkt die Resorption der Wirksubstanzmischung generell und unterstützt ebenfalls den Fettabbau.

Bitterorangenextrakt kann ebenfalls zur Gewichts- bzw. Körperfettreduktion beitragen, da es positiv auf die Thermogenese einwirkt. In einer bevorzugten Ausführung ist Trigonella foenum graecum L. in einem Anteil von 15 Gew.% bis 90 Gew.%, vorzugsweise in einem Anteil von 65 Gew.% bis 80 Gew.%, in der Wirksubstanzmischung enthalten.

Camelia sinsensis kann vorzugsweise mit einem Anteil von 5 Gew.% bis 50 Gew.%, vorzugsweise mit einem Anteil von 20 Gew.% bis 30 Gew.% in der Wirksubstanzmischung enthalten sein.

Curcumaextrakt kann in einer bevorzugten Ausgestaltung mit einem Anteil von 0,2 Gew.% bis 2,5 Gew.%, vorzugsweise mit einem Anteil von 0,7 Gew.% bis 1,3 Gew.% in der Wirksubstanzmischung enthalten sein.

Weiter kann der ayurvedische Gewürzextrakt, insbesondere der Pfefferextrakt, mit einem Anteil von 0,05 Gew.% bis 0,8 Gew.%, vorzugsweise mit einem Anteil von 0,15 Gew.% bis 0,4 Gew.%, in der Wirksubstanzmischung enthalten sein.

In einer weiter bevorzugten Ausgestaltung kann Bitterorangenextrakt (Citrus aurantium) mit einem Anteil von 0,2 Gew.% bis 4,0 Gew.%, vorzugsweise mit einem Anteil von 0,6 Gew.% bis 2,0 Gew.%, in der Wirksubstanzmischung enthalten sein.

In einer weiter bevorzugten Ausgestaltung kann eine Steviakomponente (bzw. eine süßungsäquivalenter Nateil eines Steviaextrakts oder -konzentrats) einen Anteil von 0,01 bis 2 Gew.%, vorzugsweise von 0,05 bis 1 Gew.%, weiter vorzugsweise von 0,1 bis 0,5 Gew. % haben.

In einer weiter bevorzugten Ausgestaltung ist das Nahrungsergänzungsmittelpräparat insbesondere durch Bereitstellung in Kapseln so konfektioniert, dass die Tagesdosis für Trigonella foenum-graecum L. im Bereich von 300 mg bis 5 g, vorzugsweise im Bereich von 900 mg bis 1500 mg, und/oder die Tagesdosis von Camelia sinsensis im Bereich von 200 mg bis 2500 mg, vorzugsweise von 600 mg bis 1200 mg, und/oder die Tagesdosis von Curcumaextrakt im Bereich von 2 mg bis 100 mg, vorzugsweise im Bereich von 5 mg bis 30 mg, und/oder die Tagesdosis des ayurvedischen Gewürzextrakts, insbesondere des Pfefferextrakts, im Bereich von 1 mg bis 20 mg, vorzugsweise im Bereich von 2 mg bis 6 mg, und/oder die Tagesdosis des Bitterorangenextrakts im Bereich von 5 mg bis 50 mg, vorzugsweise im Bereich von 10 mg bis 30 mg, liegt.

Bevorzugtermaßen liegt der in Camelia sinsensis enthaltene Anteil an Polyphenolen im Bereich von 5 Gew.% bis 20 Gew.%, vorzugsweise im Bereich von 8 Gew.% bis 15 Gew.%, bezogen auf die gesamte Wirksubstanzmischung. Die Tagesdosis an Polyphenolen liegt bevorzugterweise im Bereich von 75 mg bis 500 mg, vorzugsweise bei 150 mg bis 300 mg.

In einer weiter bevorzugten Ausgestaltung liegt der in Camelia sinsensis enthaltene Anteil an Koffein im Bereich von 0,5 Gew.% bis 5 Gew.%, vorzugsweise im Bereich von 1,0 Gew.% bis 2,9 Gew.%, bezogen auf die Wirksubstanz. Die Tagesdosierung von Koffein liegt bevorzugterweise im Bereich von 10 mg bis 100 mg, vorzugsweise in einem Bereich von 20 mg bis 50 mg.

In einer besonders bevorzugten Ausgestaltung ist die Wirksubstanz gegebenenfalls unter Beimengung von weiteren Träger- und Zusatzstoffen, insbesondere unter Beimengung von MCT-Öl, in oral einnehmbaren Kapseln konfektioniert. Das MCT-Öl hat insbesondere die Funktion feine Stäube zu binden und so die gesamte Wirksubstanz besser in die Kapseln abfüllen zu können.

Weiter wird ein diätisches Lebensmittel, umfassend eine vorbestimmte Anzahl von Kapseln mit dem Nahrungsergänzungsmittelpräparat nach einem der Ansprüche 1 bis 10, einem Begleitpräparat zur Entwässerung, Entsäuerung und Öffnung der Fettspeicher sowie einem Mahlzeitenersatz in Form eines oder mehrerer Shakes beansprucht. Das Begleitpräparat zur Entwässerung, Entsäuerung und Öffnung der Fettspeicher kann vorzugsweise die Mineralstoffe und Spurenelemente Vitamin D, Calcium, Magnesium, Selen, Kupfer, Zink und Chrom enthalten und weiterhin mindestens einen Katalysator und/oder Wirkstoff, vorzugsweise in Pulverform enthalten, der die Resorption der Mineralstoffe und/oder Spurenelemente steigert und/oder verbessert und/oder beschleunigt.

Als Begleitpräparat zur Entwässerung, Entsäuerung und Öffnung der Fettspeicher kann insbesondere ein Basenpulver, beispielsweise das Produkt Restorate®, in Betracht kommen. Das Basenpulver unterstützt durch die Entsäuerung den Abbau des Fettübergewichts, da es die Fettzellen aufschließt, und fördert gleichzeitig über die Entsäuerung den Abbau des Wasser-Schlackenübergewichts. Eine Gewichtsabnahme durch Entsäuerung findet relativ schnell statt und kann bei "verschlackten" Personen über Wasser-Schlackenausscheidung rasch zu hohen Gewichtsabnahmen führen. Eine ausreichende Entsäuerung verhindert auch dauerhaft die erneute Einlagerung von Wasser und Schlacken und unterstützt den Fettabbau. Je nach Übersäuerung werden ein bis drei Rationen Basenpulver pro Tag empfohlen.

Als Begleitpräparat zur Entwässerung, Entsäuerung und Öffnung der Fettspeicher kann weiterhin ein basischer Tee Anwendung finden, beispielsweise das Produkt Herbaslim®. Der basische Tee liefert bei der Gewichtsabnahme wichtige Flüssigkeit zur Neutralisation und zum Abtransport der freigesetzten Schlacken und Gifte aus den Fettzellen. Ausreichendes Trinken (mind. 1l des basischen Tees plus viel Wasser ohne Kohlensäure) verhindert damit auch auf einfache Weise mögliche "Fastennebenwirkungen" wie Kopfschmerzen oder Mundgeruch. Der basische Tee unterstützt auch in Kombination mit dem Basenpulver, die Entsäuerung und damit den Abbau des Wasser-Schlackenübergewichts.

Der Mahlzeitenersatz in Form von Shakes beinhaltet:
- wenigstens ein Sojaeiweiss, und/oder
- wenigstens einen Mikrofaser-Ballaststoff und/oder
- wenigstens einen Kohlenhydratlieferanten und/oder
- wenigstens einen Fettsäurelieferanten und/oder
- wenigstens ein fettlösliches und wenigstens ein wasserlösliches Vitamin und/oder
- wenigstens einen Mineralstoff und/oder
- wenigstens einen Geschmacksstoff und/oder
- Isoflavone und/oder
- eine Steviakomponente

Der Mahlzeitenersatz in Form eines oder mehrerer Shakes ist somit speziell für eine wirksame Gewichtsabnahme durch kalorienreduzierte Mahlzeiten mit Unterstützung der Thermogenese (Verbrennung von Kalorien in Wärme) aber auch für eine sichere und gesunde Gewichtsabnahme durch die Ausgewogenheit der wertvollen und lebenswichtigen Nähr- und Vitalstoffe konzipiert. Der Mahlzeitenersatz wird bevorzugt als Shake hergestellt.

Hochwertiges pflanzliches Sojaeiweiß mit einer sehr hohen biologischen Wertigkeit hilft die stoffwechselaktive und formgebende Muskulatur zu erhalten und damit die Gewichtsabnahme dauerhaft zu sichern. Durch den ausschließlichen Einsatz von Sojaeiweiß ist der Mahlzeitenersatz in Form eines oder mehrerer Shakes zudem frei von Milcheiweiß. Weiterhin ist der Mahlzeitenersatz lactosefrei. Dadurch ist es auch für entsprechende Allergiker verträglich.

Die Mikrofaser-Ballaststoffe bremsen den Heißhunger über einen verlangsamten Zuckereinstrom ins Blut und sorgen zudem für eine Entgiftung und eine bessere Verdauung. Die Kohlenhydrate garantieren Leistungsfähigkeit während der Gewichtsabnahme auch für das Gehirn und das Nervensystem. Darüber hinaus liefert das diätetische Lebensmittel lebensnotwendige Fettsäuren, Vitamine und Mineralstoffe. Mit Hilfe der Geschmacksstoffe werden verschiedene Geschmacksrichtungen erzeugt, so dass eine Abwechslung bei der Einnahme des Mahlzeitenersatzes entstehen kann und darüber hinaus für jede Person die richtige Geschmacksrichtung angeboten werden kann.

Isoflavone sind sekundäre Pflanzenstoffe und wirken als Phytohormone (Phytoöstrogene). Durch den Zusatz von Isoflavonen können unter anderem Wechseljahrsbeschwerden verringert, gesunde stabile Knochen gefördert sowie Osteoporose und Brustkrebs vorgebeugt werden. Darüber hinaus können Isoflavone im Zusammenhang mit dem Cholesterinspiegel den LDL-Wert erniedrigen und den HDL-Wert erhöhen sowie das Prostata-Krebs-Risiko beim Mann reduzieren. Isoflavone wirken Hormonspiegel normalisierend und dienen als Herz-Kreislauf-Schutz.

Der Mahlzeitenersatz in Form eines oder mehrerer Shakes greift speziell beim Fettübergewicht an, das längerfristig mobilisiert werden muss. Es wird empfohlen, ein bis zwei Mahlzeiten täglich durch den Mahlzeitenersatz in Form eines oder mehrerer Shakes zu ersetzen.

Insbesondere vorteilhaft ist es, wenn der Isoflavongehalt zwischen 10 mg/100 g Mahlzeitenersatz und 200 mg/100 g Mahlzeitenersatz, vorzugsweise zwischen 30 mg/100 g Mahlzeitenersatz und 60 mg/100 g Mahlzeitenersatz, insbesondere bei 33 mg/100 g Mahlzeitenersatz, liegt.

Gemäß einer beispielhaften Ausführungsform des hier vorgeschlagenen Nahrungsergänzungsmittelpräparats kann die Wirksubstanzmischung wie folgt zusammengestellt sein:
Bockshornkleesamen-Konzentrat (Trigonella foenum-graecum L.): 1.413 mg davon Ballaststoffe: 1.110 mg
Grüntee-Extrakt (Camelia sinsensis): 492 mg
davon Polyphenole: 246 mg
davon Koffein: 39 mg
Orangenfrucht (Bitter-)Konzentrat (Citrus aurantium): 25 mg
Aroma (Curcumaextrakt): 20 mg
Aroma (ayurvedischer Aromaextrakt, Pfefferextrakt): 5 mg
Stevia (getrocknet; in extrahierter bzw. konzentrierter Form in süßungsäquivalenter Menge): 5 mg
Der Wirksubstanzmischung kann noch
MCT-Öl: 10 mg
beigemischt werden.

Das hier vorgeschlagene Nahrungsergänzungsmittelpräparat eignet sich insbesondere in Kombination mit einem Begleitpräparat zur Entwässerung sowie einem Mahlzeitenersatz in Form eines oder mehrerer Shakes, um Körper- bzw. Übergewicht, insbesondere Körperfett, auf verträgliche und nachhaltige Weise abzubauen.

Das Nahrungsergänzungsmittelpräparat kann also insbesondere Beta-Carotin (Provitamin A) und/oder andere Carotinoide und/oder Vitamin C und/oder Vitamin E und/oder ein B-Vitamin, wie Vitamin B₁, Vitamin B₆ und/oder Vitamin B₁₂, und/oder Biotin und/oder Folsäure und/oder Niacin und/oder Pantothensäure und/oder Natrium und/oder Kalium und/oder Magnesium und/oder Calcium und/oder Selen und/oder Phosphor und/oder Guaranaextrakt und/oder Zink und/oder Kupfer und/oder Chrom und/oder Eisen und/oder Enzyme und/oder Milchsäurebakterien (probiotisch) und/oder Oligofructose (prebiotisch) und/oder Ballaststoffe und/oder sekundäre Pflanzenstoffe und eine Steviakomponente aufweisen.

Weiterhin können die Aminosäuren Leucin, Isoleucin, Valin, Lysin, Phenylalanin, Treonin und/oder Tryptophan vorgesehen sein.

Das folgende Ausführungsbeispiel kann als Pulver vorliegen und ist insbesondere dafür vorgesehen in Wasser zur Herstellung eines Getränks eingerührt zu werden. Beispielsweise können 7 g des Pulvers in 150 ml Wasser eingerührt werden. Das Pulver dieses Beispiels umfasst die folgenden Inhaltsstoffe (bezogen auf 7 g des Pulvers in 150 ml Wasser):

| Inhaltsstoff | Menge in einer 7-Gramm-Portion (150-Milliliter Wasser) | Grenzwerte |
|---|---|---|
| Vitamin C | 60 mg | 10 mg - 1.000 mg |
| Niacin | 15 mg | 2 mg - 200 mg |
| Vitamin E | 10 mg | 1 mg - 100 mg |
| Pantonthensäure | 5,0 mg | 0,7 mg - 50 mg |
| Vitamin B6 | 1,6 mg | 0,2 mg - 18 mg |
| Vitamin B1 | 1,4 mg | 0,2 mg - 15 mg |
| Vitamin B2 | 1,3 mg | 0,15 mg - 14 mg |
| Folsäure | 120 µg | 10 µg - 1 mg |
| Vitamin K | 40 µg | 6 µg - 500 µg |
| Biotin | 30 µg | 6 µg - 400 µg |
| Vitamin B12 | 1,0 µg | 0,1 µg - 10 µg |
| Vitamin A | 333 µg | 45 µg - 5 mg |
| Calcium | 120 mg | 20 mg - 5 g |
| Magnesium | 56 mg | 5 mg - 500 mg |
| Stevia (Steviosid) | 28 mg | 2 mg - 200 mg |

Das vorhergehende Ausführungsbeispiel ist besonders gut für Kinder und Heranwachsende geeignet. Inulin kann in einem Anteil von 4 g - 6 g, insbesondere 5 g vorliegen. Kohlenhydrate können in einem Anteil von 0,5 g - 1 g, insbesondere 0,7 g vorliegen. Der Anteil von Fett bzw. Proteinen ist vorzugsweise unter 0,1 g.

Das folgende Ausführungsbeispiel ist insbesondere auch für Erwachsene geeignet und kann als Pulver zum Auflösen in Wasser vorliegen. Eine Tagration kann beispielsweise 15 g des Pulvers in 250 ml Wasser sein und wie folgt aufgebaut sein:

| Inhaltsstoff | Menge in einer 15-Gramm-Portion (250-Milliliter Wasser) | Grenzwerte |
|---|---|---|
| Calcium | 120 mg | 10 mg - 1.000 mg |
| Magnesium | 56 mg | 5 mg - 500 mg |
| Phosphor | 120 mg | 10 mg - 1.000 mg |
| Selen | 30 µg | 3 µg - 300 µg |
| Koffein (vorzugsweise aus Guaranaextrakt bzw. | | |
| Guaranaextraktpulver) | 38 mg | 4 mg - 400 mg |
| Vitamin C | 120 mg | 15 mg - 800 mg |
| Niacin | 36 mg | 5 mg - 400 mg |
| Vitamin E | 10 mg | 1 mg - 100 mg |
| Panthonthensäure | 6 mg | 1 mg - 50 mg |
| Vitamin B6 | 2 mg | 500 µg - 15 mg |
| Vitamin B2 | 1,6 mg | 300 µg - 12 mg |
| Vitamin B1 | 1,4 mg | 200 µg - 10 mg |
| Folsäure | 200 µg | 20 µg - 2 mg |
| Vitamin B12 | 1 µg | 0,1 µg - 10 µg |
| Biotin | 150 µg | 15 µg - 2 mg |
| Vitamin A | 333 µg | 30 µg - 3 mg |
| Stevia (Steviosid) | 52 mg | 10 mg - 200 mg |

Kohlenhydrate können in einem Anteil von 10 g - 14 g, insbesondere 12 g vorliegen. Der Eiweißanteil bzw. Fettanteil ist vorzugsweise unter 0,1 g.

Ein weiteres Ausführungsbeispiel kann insbesondere für den Zellschutz zum Einsatz kommen und beispielsweise in Tagerationen von 7,5 g (Pulver) in 150 ml Wasser zum Einsatz kommen. 7, 5 g des Pulvers können die folgenden Inhaltsstoffe umfassen:

| Inhaltsstoff | Menge in einer 7,5-Gramm-Portion (150-Milliliter Wasser) | Grenzwerte |
|---|---|---|
| Selen | 30 µg | 3 µg - 300 µg |
| Vitamin C | 56 mg | 5 mg - 500 mg |
| Vitamin E | 9 mg | 1 mg - 100 mg |
| Vitamin A | 333 µg | 30 µg - 3 mg |
| Bioflavonoide (vorzugsweise aus Orangenextrakt bzw. Orangenkonzentrat ) | 50 mg | 5 mg - 500 mg |
| Stevia (Steviosid) | 34 mg | 5 mg - 100 mg |

Kohlenhydrate können in einem Anteil von 6 g - 8 g, insbesondere 7 g vorliegen. Der Fett- und/oder Proteinanteil ist vorzugsweise unter 1 g.

Ein weiteres (folgendes) Ausführungsbeispiel kann insbesondere bei einem empfindlichen Magen des Anwenders und beispielsweise in Tagerationen von 12 g (Pulver) in 200 ml Wasser zum Einsatz kommen. 12 g des Pulvers können die folgenden Inhaltsstoffe umfassen:

| Inhaltsstoff | Menge in einer 12-Gramm-Portion (200-Milliliter Wasser) | Grenzwerte |
|---|---|---|
| Selen | 30 µg | 3 µg - 300 µg |
| Vitamin C | 60 mg | 6 mg - 600 mg |
| Vitamin E | 10 mg | 1 mg - 100 mg |
| Vitamin A | 333 µg | 30 µg - 3 mg |
| Stevia (Steviosid) | 24 mg | 2 mg - 200 mg |

Kohlenhydrate können in einem Anteil von 4 g bis 6 g, insbesondere 5 g vorliegen. Der Fett- und/oder Proteinanteil ist vorzugsweise unter 1 g, insbesondere unter 0,2 g. Ballaststoffe können in einem Anteil von 4 g bis 6 g, insbesondere 5 g vorliegen.

Grundsätzlich kann im Allgemeinen zur Süßung der Nahrungsergänzungsmittelpräparate ausschließlich Stevia zum Einsatz kommen. Es ist jedoch auch denkbar die Süßungswirkung nur anteilig durch Stevia bereitzustellen und durch andere Süßungsmittel, vorzugsweise Zucker, insbesondere Fructose, zu ergänzen. Die Süßungswirkung von Stevia in Zuckeräquivalenten kann beispielsweise mindestens 20 %, vorzugsweise mindestens 50 % und/oder höchstens 80 %, vorzugsweise höchstens 60 % einer Gesamtsüße ausmachen.

Weitere Ausführungsformen ergeben sich aus den Unteransprüchen.

## Patentansprüche

1. Nahrungsergänzungsmittelpräparat, das insbesondere pulverförmig und/oder als Konzentrat vorliegt, umfassend eine Vielzahl von Nahrungsergänzungsstoffen, wie Vitamine und/oder Mineralstoffe, in einem vorbestimmten Verhältnis, sowie eine Steviakomponente.

2. Nahrungsergänzungsmittelpräparat nach Anspruch 1,
**dadurch gekennzeichnet, dass**
die Steviakomponente getrocknete Pflanzenteile der Stevia Rebaudiana, wie Blätter und/oder ein Steviaextrakt und/oder Steviosid und/oder Steviobiosid und/oder Rebaudiosid und/oder Dulcosid umfasst.

3. Nahrungsergänzungsmittelpräparat nach einem der vorhergehenden Ansprüche, **dadurch gekennzeichnet, dass**
die Steviakomponente maskiert ist, vorzugsweise durch Aufsprühen und/oder eine Verkapselung mit einem Maskierungsmittel.

4. Nahrungsergänzungsmittelpräparat nach einem der vorhergehenden Ansprüche, **dadurch gekennzeichnet, dass**
die Steviakomponete in Form von mizellierten Partikeln vorliegt.

5. Nahrungsergänzungsmittelpräparat nach einem der vorhergehenden Ansprüche, **gekennzeichnet durch**
- eine koffeinhaltige Substanz, insbesondere Guarana und/oder
- Niacin und andere B-Vitamine und/oder
- mindestens eine als Radikalfänger wirkende Substanz und/oder
- getrocknete Braunalgen oder Braunalgenextrakte, wahlweise in Form von deren Salzen.

6. Nahrungsergänzungsmittelpräparat nach einem der vorhergehenden Ansprüche, **dadurch gekennzeichnet, dass**
die Mischung in pulvriger Form zur Herstellung einer wässrigen Lösung und/oder in fester oder flüssiger Form oder als Emulsion vorliegt.

7. Nahrungsergänzungsmittelpräparat nach einem der vorhergehenden Ansprüche, zur Verwendung als Mittel zur Erhöhung/Stimulation des O₂-Antransportes in menschliche oder tierische Zellsysteme oder zur Verwendung als ein die Durchblutung förderndes Mittel oder zur Verwendung als ein die Konzentration förderndes Mittel.

8. Nahrungsergänzungsmittelpräparat nach einem der vorhergehenden Ansprüche, **gekennzeichnet durch**:
- probiotische Kulturen mit einem vorbestimmten Anteil an lebenden probiotischen Milchsäurebakterien und/oder
- einen Gewürzextrakt nach dem ayurvedischen Prinzip, beispielsweise, Maricha als Alkaloid.

9. Nahrungsergänzungsmittelpräparat nach einem der vorhergehenden Ansprüche, **dadurch gekennzeichnet, dass**
mindestens eine weitere als Radikalfänger wirkende Substanz, insbesondere Bioflavonoide, enthalten ist.

10. Nahrungsergänzungsmittelpräparat nach einem der vorhergehenden Ansprüche, **dadurch gekennzeichnet, dass**
präbiotische Fructooligosaccharide und/oder Inulin zur Förderung der probiotischen Kulturen enthalten sind.

11. Nahrungsergänzungsmittelpräparat nach einem der vorhergehenden Ansprüche, **dadurch gekennzeichnet, dass**
weiterhin eine Mehrzahl verschiedener löslicher und/oder unlöslicher Ballaststoffe enthalten ist.

12. Nahrungsergänzungsmittelpräparat nach einem der vorhergehenden Ansprüche, **dadurch gekennzeichnet, dass**
eine Mehrzahl verschiedener pflanzlicher Verdauungsenzyme enthalten ist.

13. Nahrungsergänzungsmittelpräparat nach einem der vorhergehenden Ansprüche, **gekennzeichnet durch**:
- probiotische Kulturen mit einem vorbestimmten Anteil an lebenden probiotischen Milchsäurebakterien und/oder
- ein thermogenetisch-lipolytisch-resorptionssteigerndes Agens, insbesondere Pfefferextrakt, zur Verstärkung der resorptiven Prozesse im Darm sowie ein kapillardilatives Agens, insbesondere Nicotinsäure, zur Erzielung einer Kapillardilatation im Intestinaltrakt.

14. Nahrungsergänzungsmittelpräparat nach einem der vorhergehenden Ansprüche, **dadurch gekennzeichnet, dass**
die probiotischen Kulturen zumindest zwei, vorzugsweise drei, besonders bevorzugt mindestens vier verschiedene Kulturen (Keimstämme) umfassen.

15. Nahrungsergänzungsmittelpräparat nach einem der vorhergehenden Ansprüche, **dadurch gekennzeichnet, dass**
die probiotischen Kulturen als einzige Kultur oder kombiniert zu zwei Kulturen aus der Gruppe umfassen Lactobacillus acedophilus, Lactobazillus delbrückii, insbesondere Lactobacillus bulgaricus, Bifidobaktierien, Streptokokkus thermophilus ausgewählt sind.

16. Nahrungsergänzungsmittelpräparat nach einem der vorhergehenden Ansprüche, **dadurch gekennzeichnet, dass**
weiterhin Oligofructose enthalten ist.

17. Nahrungsergänzungsmittelpräparat nach einem der vorhergehenden Ansprüche, **dadurch gekennzeichnet, dass**
- Mineralstoffe und Spurenelemente, wie Vitamin D, Kalzium, Magnesium, Selen, Kupfer, Zink und/oder Chrom, enthalten sind und/oder
- das Präparat mindestens einen Katalysator und/oder Wirkstoff, vorzugsweise in Pulverform, enthält, der die Resorption der Mineralstoffe und/oder Spurenelemente steigert und/oder verbessert und/oder beschleunigt.

18. Nahrungsergänzungsmittelpräparat nach einem der vorhergehenden Ansprüche, **dadurch gekennzeichnet, dass**
das Nahrungsergänzungsmittelpräparat Trigonella Foenum-Graecum L. (Bockshornkleesamen) und/oder Camelia sinsensis mit einem Anteil von Polyphenolen und Koffein und/oder ein Curcumaextrakt und/oder ein ayurvedisches Gewürzextrakt, insbesondere Pfefferextrakt, sowie Bitterorangenextrakt, enthält.

19. Nahrungsergänzungsmittelpräparat nach einem der vorhergehenden Ansprüche, **dadurch gekennzeichnet, dass**
das Nahrungsergänzungsmittelpräparat als Shake vorliegt oder zur Verwendung für ein Shake ausgebildet ist.

20. Nahrungsergänzungsmittelpräparat nach einem der vorhergehenden Ansprüche, **dadurch gekennzeichnet, dass**
das Nahrungsergänzungsmittelpräparat als Tee vorliegt oder zur Verwendung für einen Tee ausgebildet ist.

21. Nahrungsergänzungsmittelpräparat nach einem der vorhergehenden Ansprüche, **dadurch gekennzeichnet, dass**
das Nahrungsergänzungsmittelpräparat als Pulver, insbesondere zum Anrühren in Wasser vorliegt und einen Gewichtsanteil von mindestens 0,05 %, vorzugsweise mindestens 0,1 %, noch weiter vorzugsweise mindestens 0,3 % und/oder höchstens 1 %, vorzugsweise 0,8 %, noch weiter vorzugsweise 0,45 % enthalten ist, wobei vorzugsweise der Gewichtsanteil des Pulvers gegenüber dem Wasseranteil etwa 5 % bis 20 %, vorzugsweise 7 % bis 10 % beträgt.

22. Verwendung einer Steviakomponente für ein Nahrungsergänzungsmittelpräparat insbesondere nach einem der vorhergehenden Ansprüche, vorzugsweise zur Süßung und/oder antioxidativen Wirkung.
